# EUROPÄISCHE PATENTSCHRIFT

(11) **EP 1 372 607 B1**
(45) Veröffentlichungstag und Bekanntmachung des Hinweises auf die Patenterteilung: **03.10.2012**
(21) Anmeldenummer: 02726067.8
(22) Anmeldetag: 15.03.2002
(51) Int. Cl.: A61K 9/107, A61P 37/04

(54) **ADJUVANS-EMULSION UND DEREN VERWENDUNG**
ADJUVANT EMULSION AND METHOD OF USING SAID EMULSION
EMULSION ADJUVANTE ET PROCEDE POUR L'UTILISER

(30) Priorität: 16.03.2001 DE 10113284
(43) Veröffentlichungstag der Anmeldung: 02.01.2004
(73) Patentinhaber: Exner, Heinrich, 39291 Möckern (DE)
(72) Erfinder: Exner, Heinrich, 39291 Möckern (DE)
(74) Vertreter: Kietzmann, Manfred
(86) Internationale Anmeldenummer: PCT/DE2002/001020
(87) Internationale Veröffentlichungsnummer: WO 2002/074283

(56) Entgegenhaltungen:
- EP-A- 0 781 559
- WO-A-00/50085
- WO-A-91/00107
- WO-A-95/15768
- US-B1- 6 288 026

## Beschreibung

Die Erfindung betrifft eine Silikonölemulsion zur Verwendung als Arzneimittel bzw. zur Herstellung von Arzneimitteln.

### [Stand der Technik]

Im Patent DE 44 99 552 ist ein Adjuvans für Antigene und ein Verfahren zu seiner Herstellung und Verwendung unter Schutz gestellt. In dieser Patentschrift ist ein inkomplettes Adjuvans in Form einer Öl-in-Wasser-Emulsion beschrieben, bestehend aus
- 0,01-30% Polydimethylsiloxanen, die einen Polymerisationsgrad von n = 20-400 und eine kinematische Viskosität von 20-1000 mm²/s bei 20°C aufweisen,
- 0,01-15% Komplexemulgator mit einem HLB-Wert von 9 -16,
- 45-99% biokompatibler Salzlösung,
- 0,01-10% Dimethylsulfoxid und
- 0,0001-1% Chelatbildner.

Diesem inkompletten Adjuvans können zur Immunisierung Peptidoglykane auf der Basis von speziesspezifischen St.aureus-Stämmen und/oder anderen Stämmen in einer Konzentration von 0,00001 bis 1 mg Eiweißstickstoff pro ml Adjuvans und wasserlösliche natürliche und/oder synthetische Polymere in einer Konzentration von 0,0001 bis 10 mg pro ml Adjuvans zugesetzt werden.

Mit dieser Erfindung werden Adjuvantien zur Verfügung gestellt, die durch die Kombination mit Impfantigenen oder in Kombination mit Peptidoglycanen in einer histokompatiblen Formulierung die Abwehrmechanismen im Körper so weit stimulieren, dass neben der aktiven Immunprophylaxe auch schwacher Antigene eine allgemeine und spezifische Immuntherapie erfolgt. Mit diesem Adjuvans werden gute Erfolge bei Immuntherapien erreicht. Von Nachteil ist, dass die Herstellung des kompletten Adjuvans bzw. das Hinzufügen von Proteinen relativ aufwendig ist. Ein pathophyeiologischer Nachteil besteht in der Gefahr der anaphylaktischen Reaktion des Organismus bei mehrmaliger Applikation des kompletten Adjuvans.

Im Patent DE 44 99 552 ist auch die Verwendung des inkompletten Adjuvans für die Herstellung von Vakzinen und für die Immunisierung in der Human- und Veterinärmedizin beschrieben. Das Patent erhält jedoch keine brauchbaren Informationen für die alleinige Anwendung des inkompletten Adjuvans als Arzneimittel für konkrete Krankheiten. Bei allen in diesem Patent angeführten Beispielen wird eine Vakzination durchgeführt, bei der die Patienten durch das Impfen mit einer geschwächten Form eines Organismus gegen einen Infektionsorganismus immun gemacht werden bzw. gekeilt werden sollen.

Ein weiterer Nachteil der im Patent DE 44 99 552 angegebenen Lösung besteht darin, dass reine Öl-in-Wasser-Emulsionen im Körper schnell abgebaut werden, was deren Verabreichung in kürzeren Intervallen zur Folge hat.

Die WO-A-9515768 offenbart ein Adjuvans auf Basis einer Öl-in-Wasser-Emulsion, das aus 0,5 % Polydimethylsiloxanen, 0 35 % eine Komplexemulgators bestehend aus einem Gemisch aus hydrophoben und hydrophilen Emulgatoren mit einem HLB-Wert von 12, 0,25 % Glycerol, 98,69 % einer Salzlösung, 0,21 % Dimethylsulfoxid und 0,02 % Chelatbildner besteht. Diese Emulsion wird ohne zusätzliche Antigene für die Behandlung von Infektionskrankheiten verwendet.

Der WO-A-0050085 ist ebenfalls eine Öl-in-Wasser-Emulsion auf Basis von Silikonölen zu entnehmen, die keine Proteine enthält. Die in dieser Schrift offenbarte Emulsion besteht aus 0,01 - 30 % Polydimethylsiloxanen, 0,01 - 15 % eines Gemisches aus hydrophoben und hydrophilen Emulgatoren, 45 - 99 % einer Salzlösung und 0,01 - 10 % DMSO. Das Emulgatorgemisch kann aus 25 - 35 % Sorbitantrioleat, 25 - 35% Cetylstearylalkohol, und 35 - 45 % Polysorbat 80 zusammengesetzt sein und einen HLB-Wert von 1 bis 14 aufweisen.

### [Aufgabe der Erfindung]

Der Erfindung liegt die Aufgabe zugrunde, eine Emulsion zur intramuskulären und/oder subkutanen Applikation vorzuschlagen, die
(1) nach der Applikation lange im Körper verweilt,
(2) frei von Proteinen ist,
(3) allein oder in Kombination mit anderen intramuskulär und/oder subkutan applizierbaren Wirkstoffen, einschließlich Antigenen, verwendet wird zur Immunisierung in der Human- und Veterinärmedizin und zur Herstellung von Mitteln zur Behandlung oder Prophylaxe wenigstens einer der folgenden Indikationen: SIRS, Viruserkrankungen wie etwa Hepatitis C und AMIDS, Colitis ulcerosa, Morbus Crohn, Parkinson, Morbus Alzheimer, MS, infektiösen Hospitalismus und/oder Infektionskrankheiten viraler und/oder bakterieller Genese, Asthma bronchiale, Nieren- und Harnwegsinfektionen, Migräne, rheumatoide Arthritis, Sudeck, akuten und chronischen Entzündungen, Venen- und Arterienentzündungen, akuten und chronischen Erschöpfungszuständen, Tumorerkrankungen, Muskelschwundkrankheiten, Hämorrhoiden, CIHK, Hypertonie, Alterungsvorgängen, akuten und chronischen Schmerzzuständen, Spastiken, Polyneuropathien, Prostata Neoplasien, Haarausfall, Impotenz, Neurodermitis, Cervicalsyndrom, Gewebetraumatisierungen.

Die vorliegende Erfindung beruht auf der Feststellung, dass eine doppelte Emulsion (d.h. eine Emulsion bestehend aus einem Gemisch aus einer Wasser-in-Öl-Emulsion und einer Ö.L-in-Wasser-Emulsion) bestehend aus

| | |
|---|---|
| 0,01 - 75% | Polydimethylsiloxanen, |
| 0,01 - 15% | einem Gemisch aus hydrophoben und hydrophilen Emulgatoren mit einem HLB-Wert von 1 bis 14, |
| 0,10 - 99% | biokompatibler Salzlösung, |
| 0,01, - 20% | Dimethylsulfoxid, |
| 0,0001 - 1% | Chelatbildner und/oder |
| 0,01 - 10% | Glycerol |

zur Herstellung eines lange im Körper verweilenden Arzneimittels gegen SIRS, Viruserkrankungen wie etwa Hepatitis C und AIDS, Colitis ulcerosa, Morbus Crohn, Parkinson, Morbus Alzheimer, MS, infektiösen Hospitalismus und/oder Infektionskrankheiten viraler und/oder bakterieller Genese, Asthma bronchiale, Nieren- und Harnwegsinfektionen, Migräne, rheumatoide Arthritis, Sudeck, akuten und chronischen Entzündungen, Venen- und Arterienentzündungen, akuten und chronischen Erschöpfungszuständen, Tumorerkrankungen, Muskelschwundkrankheiten, Hämorrhoiden, CIHK, Hypertonie, Alterungsvorgängen, akuten und chronischen Schmerzzuständen, Spastiken, Polyneuropathien, Prostata Neoplasien, Haarausfall, Impotenz, Neurodermitis, Cervicalsyndrom,

Gewebetraumatisierungen bzw. zu deren Behandlung verwendet werden kann.

Die Polydimethylsiloxane weisen einen Polymerisationsgrad von n = 20-400 und eine kinematische Viskosität von 20-1300 mm²/s auf.

Der Emulgator kann aus 10-25% Sorbitanmonooleat, 10-25% Sorbitantrioleat, 10-25% Cetylstearylalkohol und 10-25% Polysorbat 80 bestehen.

Durch die Anwendung dieser erfindungsgemäßen Emulsion, die im Körper des Patienten langsamer abgebaut wird als reine Öl-in-Wasser-Emulsionen, wird auch bei großen Intervallen der Applikation ein nachhaltiger Effekt erzielt.

Bei der Behandlung mit dem erfindungsgemäßen Medikament ist die Dauer der Behandlung bis zum ersten Effekt und die gesamte Dauer der Behandlung gegenüber der Behandlung mit herkömmlichen Medikamenten deutlich verkürzt. Die Behandlungsdauer und die Dauer bis zum Eintritt eines ersten Effektes lassen sich durch die Wiederholung der Applikation innerhalb weniger Stunden und über mehrere Tage hinweg weiter verkürzen. Mit der Behandlung verbundene belastende Nebenwirkungen sind nicht beobachtet worden.

Die Applikation der erfindungsgemäßen Emulsion führt auch bei solchen Indikationen zu guten Ergebnissen, die als unheilbar gelten oder bei denen der Krankheitsverlauf mit herkömmlichen Medikamenten lediglich verzögert werden kann. Das gilt insbesondere für Hepatitis C, Parkinson, Morbus Alzheimer, MS, Tumorerkrankungen, Prostata Neoplasien. Bei Patienten, blei denen die Applikation herkömmlicher Medikamente weder Heilung noch Linderung brachte und die als austherapiert galten, ist durch die Applikation des erfindungsgemäßen Medikamentes eine deutliche Verbesserung der Gesundheit und des Gesamtbefindens eingetreten.

Weiterhin hat sich gezeigt, dass die Kombination der erfindungsgemäßen Emulsion mit anderen parenteral applizierbaren Wirkstoffen zu überraschenden Effekten führt. Wird die erfindungsgemäße Emulsion mit wenigstens einem Wirkstoffe aus den Wirkstoffgruppen Antibiotika, Kortikoide, Antiparkinsonmittel, Mittel gegen Multiple Sklerose, Neuro- und Psychopharmaka, Immunstimulantien und Schmerzmittel kombiniert, dann reduziert sich die Medikation dieser Wirkstoffe gegenüber der üblichen Medikation ohne Kombination mit der erfindungsgemäßen Emulsion um bis zu 50% und in Einzelfällen um mehr als 60%. Auf diese Weise lassen sich bei voller oder gesteigerter Wirksamkeit dieser Wirkstoffe deren Nebenwirkungen auf ein Minimum reduzieren.

## Patentansprüche

1. Adjuvans-Emulsion, bestehend aus
| | |
|---|---|
| 0,01 - 75% | Polydimethylsiloxanen; |
| 0,01 - 15% | einem Gemisch aus hydrophoben und hydrophilen Emulgatoren mit einem HLB-Wert von 1 bis 14; |
| 0,10 - 99% | biokompatibler Salzlösung, |
| 0,01 - 20% | Dimethylsulfoxid, |
| 0,0001 - 1% | Chelatbildner und/oder |
| 0,01 - 10% | Glycerol, |
wobei die Adjuvans-Emulsion eine doppelte Emulsion ist.

2. Adjuvans-Emulsion nach Anspruch 1, **dadurch gekennzeichnet, dass** der Emulgator aus 10 - 25% Sorbitanmonooleat, 10 - 25% Cetylstearylalkohol, 10 - 25% Sorbitantrioleat und 10 - 25% Polysorbat 80 besteht.

3. Adjuvans-Emulsion nach Anspruch 1, **dadurch gekennzeichnet, dass** die Emulsion aus folgenden Stoffen mit den folgenden Massenanteilen in g/ml gebildet wird :
| | |
|---|---|
| 0, 01 - 0,8 | Polydimethylsiloxane, die einen Polymerisationsgrad von n = 20 - 400 und eine kinematische Viskosität von 20-1300 mm²/s aufweisen, |
| 0,01 - 0,5 | Dimethylsulfoxid, |
| 0,03 - 0,8 | Emulgator, |
| 0,25 - 0,95 | isotonische Kochsalzlösung. |

4. Adjuvans-Emulsion nach Anspruch 3, **dadurch gekennzeichnet, dass** die Emulsion EDTA Na/Ca Salz in einem Massenanteil von 0,0001 - 0,01 g/ml enthält.

5. Adjuvans-Emulsion nach den Ansprüchen 1 bis 4, **dadurch gekennzeichnet, dass** die Emulsion mit wenigstens einem der folgenden parenteral applizierbaren Wirkstoffe kombiniert wind: Antibiotika, Kortikoide, Antiparkinsonmittel, Mittel gegen Multiple Sklerose, Neuro- und Psychopharmaka, Immunstimulantien, Schmerzmittel, Antigene.

6. Verwendung der Adjuvans-Emulsion nach einem der Ansprüche 1 bis 5 zur Herstellung eines Mittels zur Immu:nisierung in der Human- und Veterinärmedizin.

7. Verwendung der Adjuvans-Emulsion nach einem der Ansprüche 1 bis 5 zur Herstellung von Mitteln zur Behandlung oder Prophylaxe wenigstens einer der folgenden Indikationen: SIRS, Viruserkrankungen wie etwa Hepatitis C und AIDS, Colitis ulcerosa, Morbus Crohn, Parkinson, Morbus Alzheimer, MS, infektiösen Hospitalismus und/oder Infektionskrankheiten viraler und/oder bakterieller Genese, Asthma bronchiale, Nieren- und Harnwegsinfektionen, Migräne, rheumatoide Arthritis, Sudeck, akuten und chronischen Entzündungen, Venen- und Arterienentzündungen, akuten und chronischen Erschöpfungszuständen, Tumorerkrankungen, Muskelschwundkrankheiten, Hämorrhoiden, CIHK, Hypertonie, Alterungsvorgängen, akuten und chronischen Schmerzzuständen, Spastiken, Polyneuropathien, Prostata Neoplasien, Haarausfall, Impotenz, Neurodermitis, Cervicalsyndrom, Gewebetraumatisierungen.

## Claims

1. Adjuvant emulsion consisting of
| | |
|---|---|
| 0.01 - 75% | polydimethylsiloxanes, |
| 0.01 - 15% | a mixture of hydrophobic and hydrophilic emulsifiers with a HLB value of 1 to 14, |
| 0.10 - 99% | biocompatible saline solution, |
| 0.01 - 20% | dimethyl sulfoxide, |
| 0.0001 - 1% | chelating agents and/or |
| 0.01 -10% | glycerol, |
wherein the adjuvant emulsion is a double emulsion.

2. Adjuvant emulsion according to claim 1, **characterized in that** the emulsifier consists of 10 - 25% sorbitan monooleate, 10 - 25% cetyl stearyl alcohol, 10 - 25% sorbitan trioleate, and 10 - 25% polysorbate 80.

3. Adjuvant emulsion according to claim 1, **characterized in that** the emulsion is formed from the following substances with the following mass fractions in g/ml:
| | |
|---|---|
| 0.01 - 0.8 | polydimethylsiloxanes with a degree of polymerization of n = 20 - 400 and a kinematic viscosity of 20 - 1300 mm²/s, |
| 0.01 - 0.5 | dimethyl sulfoxide, |
| 0.03 - 0.8 | emulsifier, |
| 0.25 - 0.95 | isotonic salt solution. |

4. Adjuvant emulsion according to claim 3, **characterized in that** the emulsion contains EDTA Na/Ca salt with a mass fraction of 0.0001 - 0.01 g/ml.

5. Adjuvant emulsion according to claims 1 to 4, **characterized in that** the emulsion is combined with at least one of the following agents that can be administered parenterally: antibiotics, corticoids, agents for Parkinson's disease, agents for multiple sclerosis, neuro- and psychotherapeutic drugs, immunostimulants, analgesics, antigens.

6. Use of the adjuvant emulsion according to any one of claims 1 to 5 for the preparation of an agent for immunization in human and veterinary medicine.

7. Use of the adjuvant emulsion according to any one of claims 1 to 5 for the preparation of agents for the therapy or prophylaxis of at least one of the following indications: SIRS, viral diseases such as hepatitis C and AIDS, ulcerous colitis, Crohn's disease, Parkinson's disease, Alzheimer's disease, MS, infectious hospitalism and/or infectious diseases of viral and/or bacterial genesis, bronchial asthma, renal and urinary tract infections, migraine, rheumatoid arthritis, Sudeck's atrophy, acute and chronic inflammations, venous and arterial inflammations, acute and chronic exhaustion, tumor diseases, muscular atrophy, hemorrhoids, chronic ischemic heart disease, hypertension, aging processes, acute and chronic pain, spasticities, polyneuropathies, prostate neoplasias, alopecia, impotence, neurodermatitis, cervical syndrome, tissue traumas.

## Revendications

1. Adjuvant-émulsion, composé de
| | |
|---|---|
| 0,01 - 75% | de polydiméthylsiloxanes ; |
| 0,01 - 15% | d'un mélange d'émulsifiants hydrophobes et hydrophiles avec une balance hydrophile/lipophile de 1 à 14 ; |
| 0,10 - 99% | de solution saline biocompatible, |
| 0,01 - 20% | de diméthylsulfoxyde, |
| 0,0001 - 1% | d'agent chélateur et/ou |
| 0,01 - 10% | de glycérol, |
l'adjuvant émulsion étant une émulsion double.

2. Adjuvant-émulsion selon la revendication 1, **caractérisé en ce que** l'émulsifiant se compose de 10 - 25% de monooléate de sorbitane, de 10 - 25% d'alcool cétylstéarylique, de 10 - 25% de trioléate de sorbitane et de 10 - 25% de polysorbate 80.

3. Adjuvant-émulsion selon la revendication 1, **caractérisé en ce que** l'émulsion est formée des substances suivantes ayant les fractions massiques suivantes en g/ml :
| | |
|---|---|
| 0,01 - 0,8 | de polydiméthylsiloxanes qui présentent un degré de polymérisation de n = 20 - 400 et une viscosité cinématique de 20 - 1300 mm²/s, |
| 0,01 - 0,5 | de diméthylsulfoxyde, |
| 0,03 - 0,8 | d'émulsifiant, |
| 0,25 - 0,95 | de solution de chlorure de sodium isotonique. |

4. Adjuvant-émulsion selon la revendication 3, **caractérisé en ce que** l'émulsion contient EDTA sel Na/Ca dans une fraction massique de 0,0001 - 0,01 g/ml.

5. Adjuvant-émulsion selon les revendications 1 à 4, **caractérisé en ce que** l'émulsion est combinée avec au moins une des substances actives suivantes administrables par voie parentérale : antibiotiques, corticoïdes, antiparkinsoniens, agents anti-sclérose en plaques, substances neurotropes et psychotropes, stimulants immunitaires, analgésiques, antigènes.

6. Utilisation de l'adjuvant-émulsion selon une des revendications 1 à 5 pour la fabrication d'un produit destiné à l'immunisation dans la médecine humaine et vétérinaire.

7. Utilisation de l'adjuvant-émulsion selon une des revendications 1 à 5 pour la fabrication de produits destinés à traiter ou à prévenir au moins une des indications suivantes :
SIRS, maladies virales comme par exemple l'hépatite B et le SIDA, colite ulcéreuse, maladie de Crohn, maladie de Parkinson, maladie d'Alzheimer,
sclérose en plaques, maladies infectieuses nosocomiales et/ou maladies infectieuses d'origine virale et/ou bactérienne, asthme bronchique, infections rénales et des voies urinaires, migraines, arthrite rhumatoïde, maladie de Sudeck,
inflammations aiguës et chroniques, phlébites et artérites, états d'épuisement aigus et chroniques, maladies tumorales, maladies amyotrophiques, hémorroïdes,
cardiopathie ischémique chronique, hypertonie, processus de vieillissement, états douloureux aigus et chroniques, pathologies spastiques, polynévropathies,
tumeurs de la prostate, chute des cheveux, impuissance, névrodermite, syndrome cervical, traumatismes cellulaires.
